# EUROPEAN PATENT APPLICATION

(11) **EP 4 700 028 A1**
(43) Date of publication of application: **25.02.2026**
(21) Application number: 24792237.0
(22) Date of filing: 17.04.2024
(51) Int. Cl.: C07D 498/04, C07D 519/00, H10K 50/10

(54) **CARBAZOLE COMPOUND AND ORGANIC ELECTROLUMINESCENT ELEMENT**

(30) Priority: 18.04.2023 KR 20230050881
(71) Applicant: Hodogaya Chemical Co., Ltd., Tokyo, 105-0021 (JP)
(72) Inventor: KASE, Kouki, Tokyo (JP); YANG, Byung-Sun, Tokyo (JP); CHA, Hyun-Wook, Tokyo (JP); KIM, Hee-Jae, Tokyo (JP); HIRAYAMA, Yuta, Tokyo (JP); HAYASHI, Shuichi, Tokyo (JP)
(74) Representative: dompatent
(86) International application number: PCT/IB2024/053725
(87) International publication number: WO 2024/218664

(57) **Abstract**

(Objective) The objective of the present invention is to provide a compound having a high refractive index in the wavelength range of 450 nm ~ 750 nm and a low extinction coefficient in the capping layer in order to improve light extraction efficiency of an organic EL element.

(Solution) The present invention focuses on the fact that a specific carbazole compound has excellent thin film stability and durability, and its refractive index can be improved by adjusting the molecular structure, and by designing a molecule and using it as a material for a capping layer, obtains an organic EL element having excellent luminous efficiency.

## Description

### [TECHNICAL FIELD]

The present invention relates to a compound suitable for a self-luminous electronic element preferable for various display devices, specifically to a carbazole compound suitable for an organic electroluminescent element (hereinafter referred to as "organic EL element"), and to organic EL elements, electronic devices, and electronic equipment using such compound.

### [BACKGROUND TECHNOLOGY]

Since an organic EL element is a self-luminous element, it is brighter than a liquid crystal element, offer excellent visibility, and enable clear displays, and therefore, active research has been conducted on it.

In 1987, C. W. Tang and others at Eastman-Kodak made organic EL elements using organic materials practical by developing a layered structure element in which various roles were assigned to each material. They stacked a phosphor capable of transporting electrons and an organic material capable of transporting holes, and injected charges of both directions into the phosphor layer to emit light, thereby obtaining high luminance of 1000 cd/m² or more at a voltage of 10 V or less (see, e.g., Patent Documents 1 and 2).

To date, many improvements have been made for the practical use of organic EL elements, and the roles of each layer of the laminated structure have been further subdivided, so that high efficiency and durability is achieved by a light-emitting element in which the anode, hole injection layer, hole transport layer, light-emitting layer, electron transport layer, electron injection layer, and cathode are sequentially formed on a substrate, and that has a bottom-emission structure emitting light from the bottom portion (see, e.g., Non-Patent Document 1).

Recently, light-emitting elements using a high-work-function metal as the anode and having a top-emission structure that emits light from the top have come to use. In a bottom-emission structure, which extracts light from the bottom portion containing a pixel circuit, the area of the light-emitting portion is limited, while in a light-emitting element with a top-emission structure, light is extracted from the top portion, so that the pixel circuit is not blocked from view, allowing for a wider light-emitting portion. In a light-emitting element with a top-emission structure, a translucent electrode, such as LiF/Al/Ag (see, e.g., Non-Patent Document 2), Ca/Mg (see, e.g., Non-Patent Document 3), or LiF/MgAg, is used as the cathode.

In such a light-emitting element, if light emitted from the light-emitting layer enters another film at an angle exceeding a certain threshold, it is totally internally reflected at the interface between the light-emitting layer and the other film, and consequently, only a portion of the emitted light can be utilized. Recently, to improve light extraction efficiency, a light-emitting element that forms a "capping layer" with a high refractive index outside a translucent electrode with a low refractive index has been proposed (see, e.g., Non-Patent Documents 2 and 3).

As an effect of the capping layer in a light-emitting element with a top-emission structure, a light-emitting element using Ir(ppy)₃ as the light-emitting material exhibited a current efficiency of 38 cd/A without a capping layer, whereas a light-emitting element using a 60-nm-thick ZnSe as the capping layer exhibited a current efficiency of 64 cd/A, an approximately 1.7-fold improvement in efficiency. Furthermore, it has been shown that the maximum transmittance and maximum efficiency of the translucent electrode and capping layer do not necessarily coincide, indicating that the maximum light extraction efficiency is determined by interference effects (see, e.g., Non-Patent Document 3).

Conventionally, the use of high-precision metal masks has been proposed for forming capping layers. However, when used under high-temperature conditions, heat-induced deformation of the metal mask can lead to reduced alignment accuracy. Therefore, ZnSe, with its high melting point of 1100°C or higher, cannot be deposited accurately at the exact position on high-precision metal masks, potentially adversely affecting the light-emitting element (see e.g., Non-Patent Document 3). Furthermore, even in the formation of film using a sputtering method, capping layers composed of inorganic materials are not suitable due to their adverse effects on the light-emitting element.

In addition, the use of tris(8-hydroxyquinoline)aluminum (hereinafter referred to as "Alq₃") as a capping layer for adjusting the refractive index has also been proposed (see e.g., Non-Patent Document 2). However, Alq₃ is known as an organic EL material commonly used as a green light-emitting material or electron transport material, and has weak absorption near 450 nm, which is close to the emission wavelength of blue light-emitting materials, and therefore, in the case of blue light-emitting elements, there were also problems such as reduced color purity and reduced light extraction efficiency.

To improve the element characteristics and significantly enhance light extraction efficiency of organic EL elements, a capping layer material with a high refractive index, low extinction coefficient, and excellent thin film stability and durability is required.

### [Prior Art Documents]

### [Patent Documents]

(Patent Document 1) US5792557
(Patent Document 2) US5639914
(Patent Document 3) WO2014/009310
(Patent Document 4) KR2164767

### [Non-Patent Documents]

(Non-Patent Document 1) 9th Seminar of the Society of Applied Physics, training session notice, pp. 55-61 (2001)
(Non-Patent Document 2) Appl. Phys. Let., 78, 544 (2001)
(Non-patent Document 3) Appl. Phys. Let., 82, 466 (2003)
(Non-patent Document 4) Tetrahedron, 58, 9633 (2002)
(Non-patent Document 5) Appl. Phys. Let., 98, 083302 (2011)

### [DESCRIPTION OF THE INVENTION]

### [TECHNICAL PROBLEM]

An object of the present invention is to provide a compound having a high refractive index in the wavelength range of 450 nm to 750 nm and a low extinction coefficient, which is preferable as a material for a capping layer of an organic EL element, and further, to provide an organic EL element having improved light extraction efficiency by using said compound.

The physical properties of a compound suitable for a capping layer of an organic EL element include (1) having a high refractive index, (2) having a low extinction coefficient, (3) being able to be deposited, (4) having stability in a thin film state, and (5) having a high glass transition temperature. Furthermore, the physical properties of the organic EL element the present invention aims to provide include (1) having high light extraction efficiency, (2) having no degradation in color purity, (3) being able to transmit light without change over time, and (4) having a long lifetime.

### [SOLUTION TO PROBLEM]

Therefore, to achieve the above objectives, the inventors of the present invention focused on the excellent thin film stability and durability of a carbazole compound and, by optimizing the molecular design, developed a material with a high refractive index in the wavelength range of 450 nm to 750 nm and a low extinction coefficient. Furthermore, by fabricating an organic EL element using the compound and conducting thorough evaluations of the characteristics of the element, the present invention was completed, as it was able to solve the problems of the conventional technology.

In other words, according to the present invention, a carbazole compound represented by the following general formula (A) or (B) and an organic EL element are provided.
1) A carbazole compound represented by the following general formula (A) or (B).

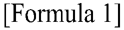

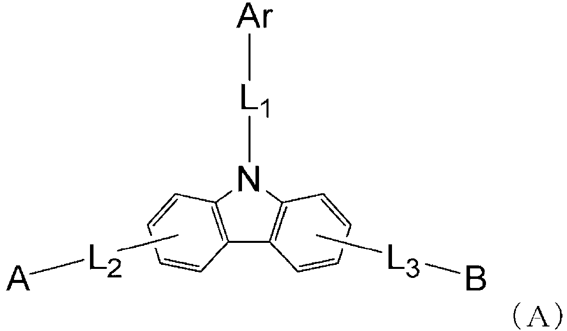

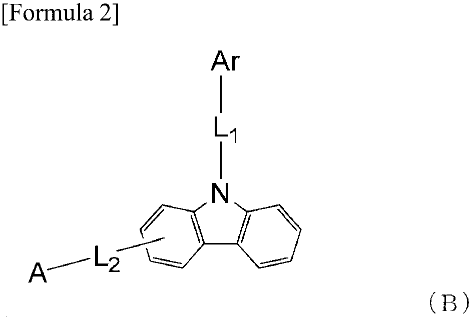

In the general formulas (A) and (B), Ar, A, and B each indicate:
a substituted or unsubstituted aromatic hydrocarbon group,
a substituted or unsubstituted aromatic heterocyclic group,
or a substituted or unsubstituted condensed polycyclic aromatic group,
with the proviso that at least one of A and B is
a substituted or unsubstituted oxazolopyridyl group,
or a substituted or unsubstituted oxazolopyrazyl group.
L₁ to L₃ are,
   a single bond,
   an unsubstituted divalent aromatic hydrocarbon group,
   an unsubstituted divalent aromatic heterocyclic group,
   or an unsubstituted divalent condensed polycyclic aromatic group.

2) The carbazole compound described in 1), represented by the following general formula (C) or (D).

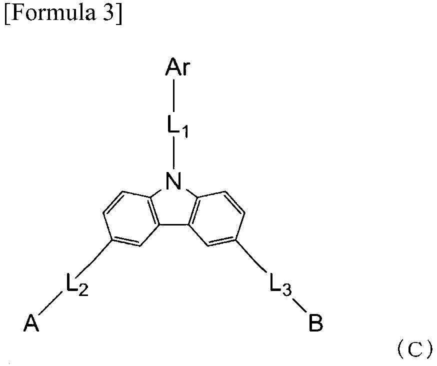

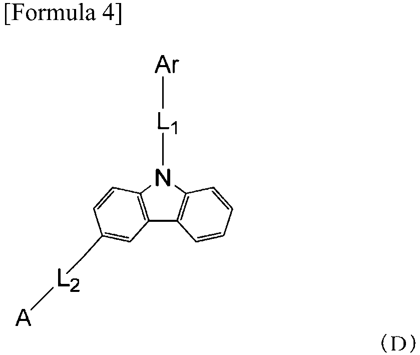

In the formula (C) or (D), Ar, A, and B and L₁ to L₃ are as defined in the above general formula (A) or (B).

3) The carbazole compound described in 2), wherein L₁ to L₃ in the general formula (C) or (D) are a single bond, an unsubstituted phenylene group, or an unsubstituted naphthylene group.

4) The carbazole compound described in 3), wherein L₁ to L₃ in the general formula (C) or (D) are a single bond, an unsubstituted 1,4-phenylene group, or an unsubstituted 2,6-naphthylene group.

5) The carbazole compound described in 4), wherein Ar, A, and B, in the general formula (C) or (D) are a substituted or unsubstituted phenyl group, a substituted or unsubstituted naphthyl group, a substituted or unsubstituted quinolyl group, a substituted or unsubstituted isoquinolyl group, a substituted or unsubstituted quinoxalyl group, a substituted or unsubstituted phenanthrenyl group, a substituted or unsubstituted phenantronyl group, a substituted or unsubstituted dibenzofuranyl group, a substituted or unsubstituted dibenzothienyl group, a substituted or unsubstituted benzoxazolyl group, a substituted or unsubstituted benzothiazolyl group, a substituted or unsubstituted benzofuranyl group, a substituted or unsubstituted benzothienyl group, a substituted or unsubstituted oxazolopyridyl group, or a substituted or unsubstituted oxazolopyrazyl group.

6) The carbazole compound described in 5), wherein Ar in the general formula (C) or (D) is an unsubstituted phenyl group, an unsubstituted naphthyl group, an unsubstituted quinolyl group, an unsubstituted isoquinolyl group, an unsubstituted quinoxalyl group, an unsubstituted phenanthrenyl group, an unsubstituted phenantronyl group, an unsubstituted dibenzofuranyl group,
an unsubstituted dibenzothienyl group, an unsubstituted benzoxazolyl group, an unsubstituted benzothiazolyl group, an unsubstituted benzofuranyl group, an unsubstituted benzothienyl group, an unsubstituted oxazolopyridyl group, or an unsubstituted oxazolopyrazyl group.

7) The carbazole compound described in 6), wherein at least one of A and B in the general formula (C) or (D) is a substituted or unsubstituted 2-oxazolopyridyl group, a substituted or unsubstituted 5-oxazolopyridyl group, or a substituted or unsubstituted 2-oxazolopyrazyl group.

8) The carbazole compound described in 7), wherein A and B in the general formula (C) or (D) are a substituted or unsubstituted 2-oxazolopyridyl group, a substituted or unsubstituted 5-oxazolopyridyl group, or a substituted or unsubstituted 2-oxazolopyrazyl group.

9) An organic EL element having at least an anode electrode, a hole transport layer, a light-emitting layer, an electron transport layer, a cathode electrode, and a capping layer in that order, wherein the capping layer contains a carbazole compound represented by the general formula (A) or (B) described in 1).

10) The organic EL element described in 9), characterized in that when the capping layer described in 9) is formed to a thickness of 30 nm to 120 nm, the refractive index of the capping layer is 1.70 or more when the transmitted light is in the wavelength range of 450 nm to 750 nm.

11) The organic EL element described in 9), wherein the capping layer is a laminate or mixed layer composed of two or more types of compounds, in which at least one type of compound is a carbazole compound represented by the general formula (A) or (B).

12) An electronic equipment or electronic element having a pair of electrodes and at least one organic layer sandwiched between them, wherein the organic layer contains a carbazole compound represented by the general formula (A) or (B) described in 1).

In "a substituted or unsubstituted aromatic hydrocarbon group," "a substituted or unsubstituted aromatic heterocyclic group," or "a substituted or unsubstituted condensed polycyclic aromatic group" that Ar, A, and B indicate in the general formula (A) or (B), "aromatic hydrocarbon group," "aromatic heterocyclic group," or "condensed polycyclic aromatic group" may include, specifically, a group selected from an aryl group having 6 to 30 carbon atoms, or a heteroaryl group having 2 to 20 carbon atoms, such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, an oxazolopyridyl group, an oxazolopyrazyl group, a quinoxalinyl group, a quinazolinyl group, a benzoimidazolyl group, a pyrazolyl group, a dibenzofuranyl group, a dibenzothienyl group, a naphthyridinyl group, a phenantronyl group, an acridinyl group, and a carbolinyl group.

In "a substituted or unsubstituted divalent aromatic hydrocarbon group," "a substituted or unsubstituted divalent aromatic heterocyclic group," or "a substituted or unsubstituted divalent condensed polycyclic aromatic group" that L₁ to L₃ indicate in the general formula (A) or (B), "divalent aromatic hydrocarbon group," "divalent aromatic heterocyclic group," or "divalent condensed polycyclic aromatic group" may include a divalent group having one hydrogen atom is removed from "an aromatic hydrocarbon group," "an aromatic heterocyclic group," or "a condensed polycyclic aromatic group" that Ar, A, and B indicate in the general formula (A), for example, a divalent group having one hydrogen atom removed from the specific groups exemplified above.

In "a substituted aromatic hydrocarbon group," "a substituted aromatic heterocyclic group," or "a substituted condensed polycyclic aromatic group" that Ar, A, and B indicate in the general formula (A) or (B), "a substituent" may include, specifically, a deuterium atom, a cyano group, a nitro group;
halogen atoms such as fluorine atom, chlorine atom, bromine atom, and iodine atom;
silyl groups such as trimethylsilyl group and triphenylsilyl group;
straight-chain or branched alkyl groups having 1 to 6 carbon atoms, such as a methyl group, an ethyl group, and a propyl group;
straight-chain or branched alkyloxy groups having 1 to 6 carbon atoms, such as a methyloxy group, an ethyloxy group, and a propyloxy group;
alkenyl groups such as a vinyl group and an allyl group;
aryloxy groups such as a phenyloxy group and a tolyloxy group;
arylalkyloxy groups such as a benzyloxy group and a phenethyloxy group;
aromatic hydrocarbon groups or condensed polycyclic aromatic groups such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, and a triphenylenyl group;
an aryl group having 6 to 30 carbon atoms, or a heteroaryl group having 2 to 20 carbon atoms such as a pyridyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a quinazolinyl group, a benzoimidazolyl group, a pyrazolyl group, a dibenzofuranyl group, a dibenzothienyl group, a carbolinyl group, a phenantronyl group, etc., and these substituents may further be substituted by the substituents exemplified above. Further, the benzene rings substituted with these substituents, or the multiple substituents substituted on the same benzene ring, may be bonded to each other to form a ring by interposing a single bond, a substituted or unsubstituted methylene group, an oxygen atom, or a sulfur atom.

In the general formula (A) or (B), L₁ to L₃ are preferably a single bond, or an unsubstituted 1,4-phenylene group, and more preferably L₁ is a single bond.

In the general formula (A) or (B), Ar is preferably an unsubstituted phenyl group or an unsubstituted naphthyl group, and more preferably an unsubstituted naphthyl group.

In the general formula (A) or (B), A and B are preferably a substituted or unsubstituted oxazolopyridyl group, and more preferably a substituted or unsubstituted 2-oxazolopyridyl group or a substituted or unsubstituted 5-oxazolopyridyl group.

In the carbazole compound of the present invention represented by the general formula (C) or (D), Ar, A, B, and L₁ to L₃ are as defined in the general formula (A) or (B), and specific examples and preferable examples of the substituents represented by each are also as described above.

In the organic EL element, the thickness of the capping layer is preferably in the range of 30 nm to 120 nm, and more preferably in the range of 40 nm to 80 nm.

### [EFFECT OF THE INVENTION]

The carbazole compound of the present invention (1) has a high refractive index in the wavelength range of 450 nm to 750 nm, (2) has a low extinction coefficient, (3) can be deposited, (4) has stability in a thin film state, and (5) has high heat resistance, and therefore, when formed as a capping layer outside of a transparent or translucent electrode of an organic EL element, the light extraction efficiency can be significantly improved.

### [DESCRIPTION OF DRAWINGS]

Figure 1 is a drawing showing the structures of Compounds 1 to 12 as examples of the carbazole compound of the present invention.
Figure 2 is a drawing showing the structures of Compounds 13 to 27 as examples of the carbazole compound of the present invention.
Figure 3 is a drawing showing the structures of Compounds 28 to 38 as examples of the carbazole compound of the present invention.
Figure 4 is a drawing showing the structures of Compounds 39 to 50 as examples of the carbazole compound of the present invention.
Figure 5 is a drawing showing the structures of Compounds 51 to 65 as examples of the carbazole compound of the present invention.
Figure 6 is a drawing showing the structures of Compounds 66 to 79 as examples of the carbazole compound of the present invention.
Figure 7 is a drawing showing the structures of Compounds 80 to 89 as examples of the carbazole compound of the present invention.
Figure 8 is a drawing showing the structures of Compounds 90 to 100 as examples of the carbazole compound of the present invention.
Figure 9 is a drawing showing the structures of Compounds 101 to 111 as examples of the carbazole compound of the present invention.
Figure 10 is a drawing showing the structures of Compounds 112 to 121 as examples of the carbazole compound of the present invention.
Figure 11 is a drawing showing the structures of Compounds 122 to 135 as examples of the carbazole compound of the present invention.
Figure 12 is a drawing showing the structures of Compounds 136 to 148 as examples of the carbazole compound of the present invention.
Figure 13 is a drawing showing the structures of Compounds 149 to 160 as examples of carbazole compound of the present invention.
Figure 14 is a drawing showing the structures of Compounds 161 to 171 as examples of the carbazole compound of the present invention.
Figure 15 is a drawing showing an example of a configuration of the organic EL element of the present invention.

### [BEST MODE FOR CARRYING OUT THE INVENTION]

Although the carbazole compounds of the present invention are novel compounds, these compounds can be synthesized, for example, by a coupling reaction using a known palladium catalyst or the like (see, e.g., Non-Patent Document 4).

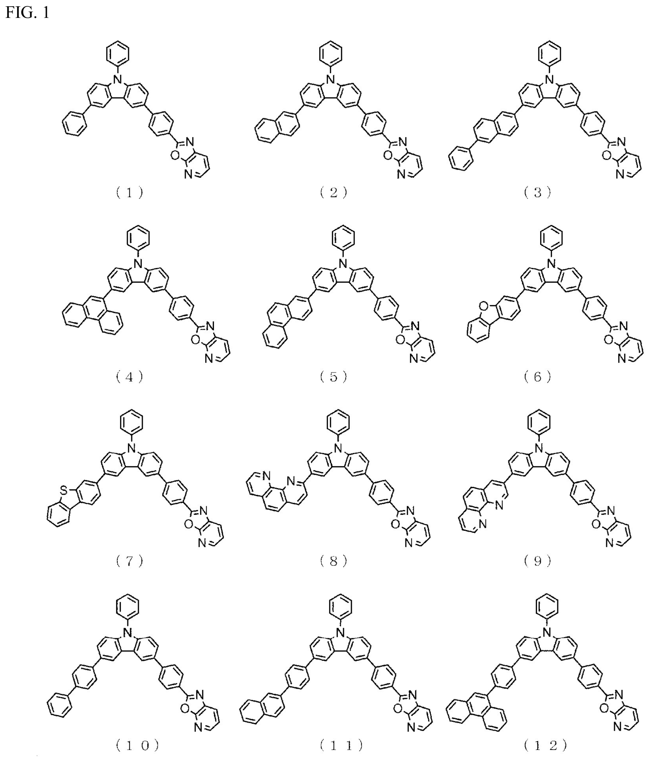

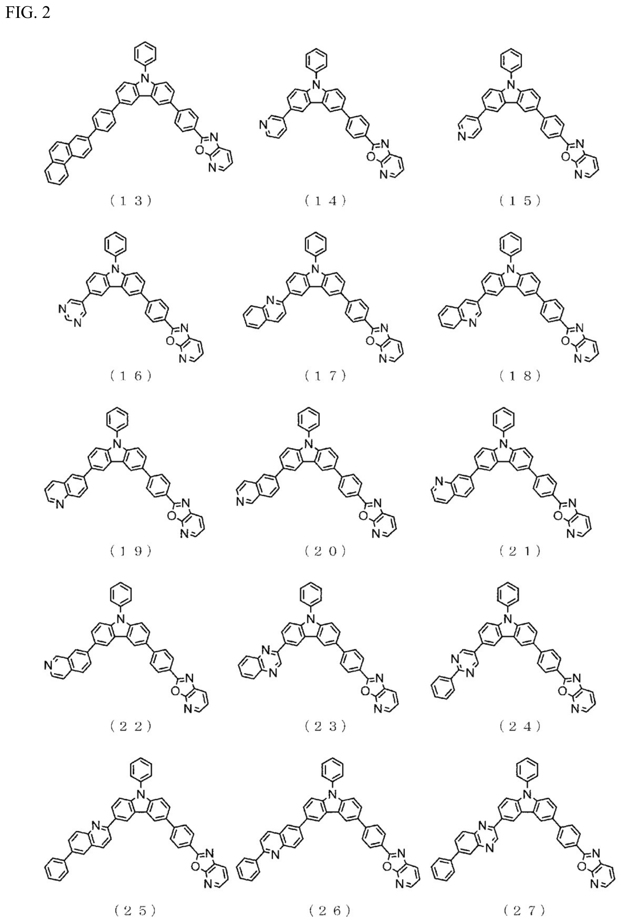

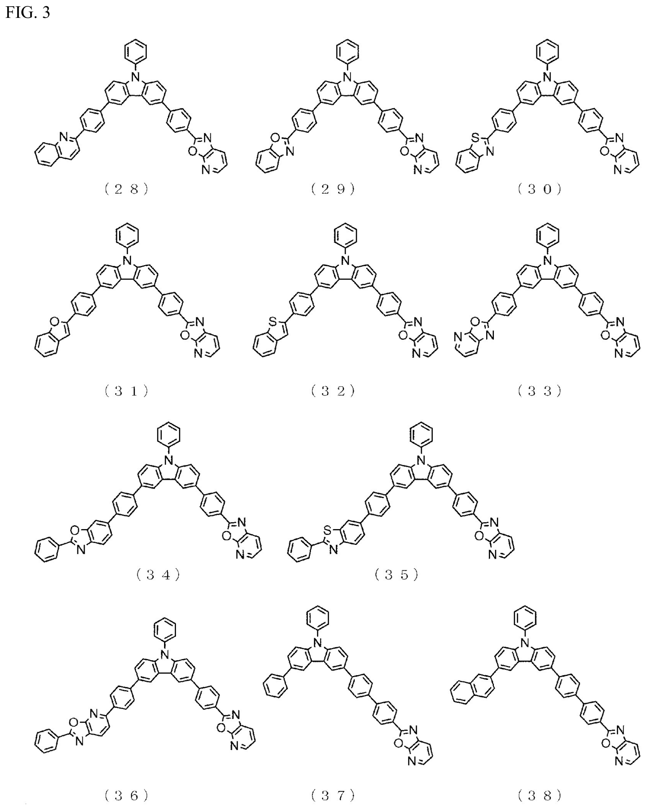

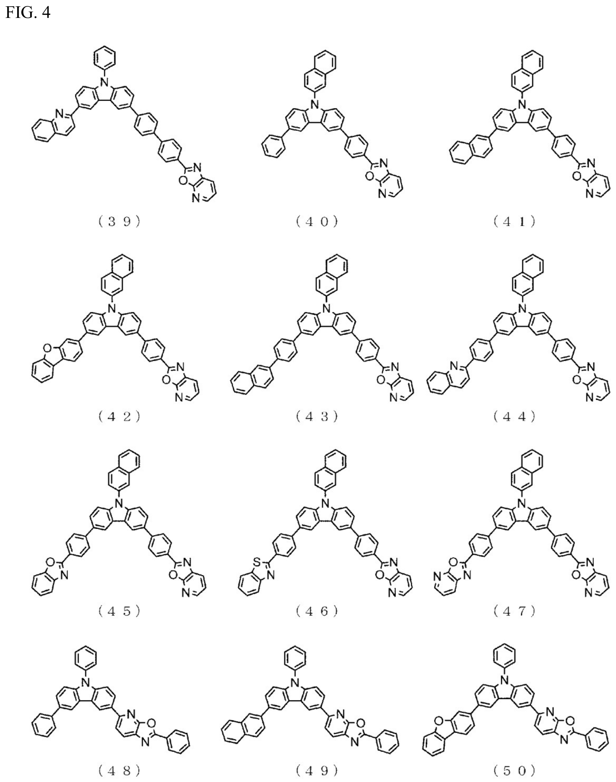

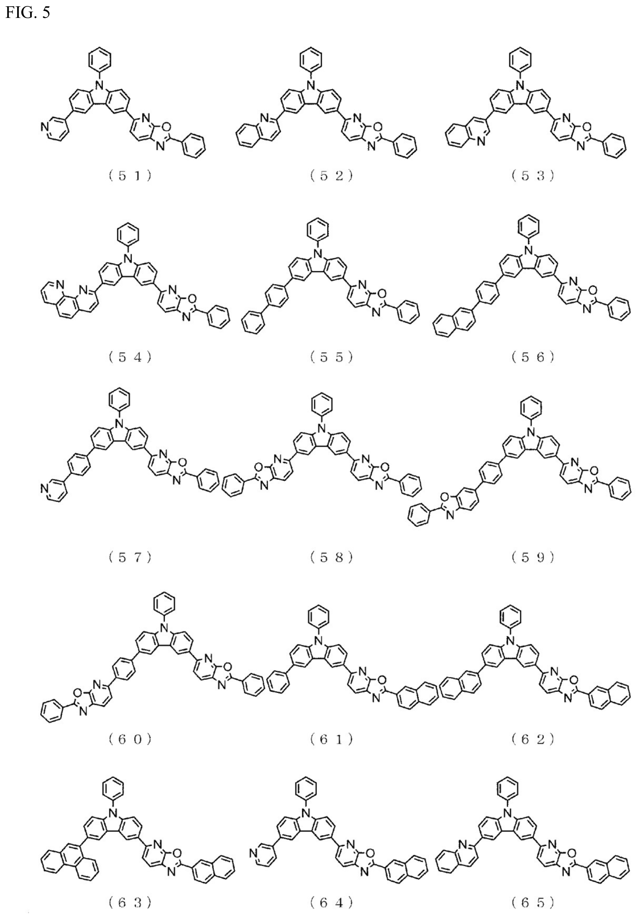

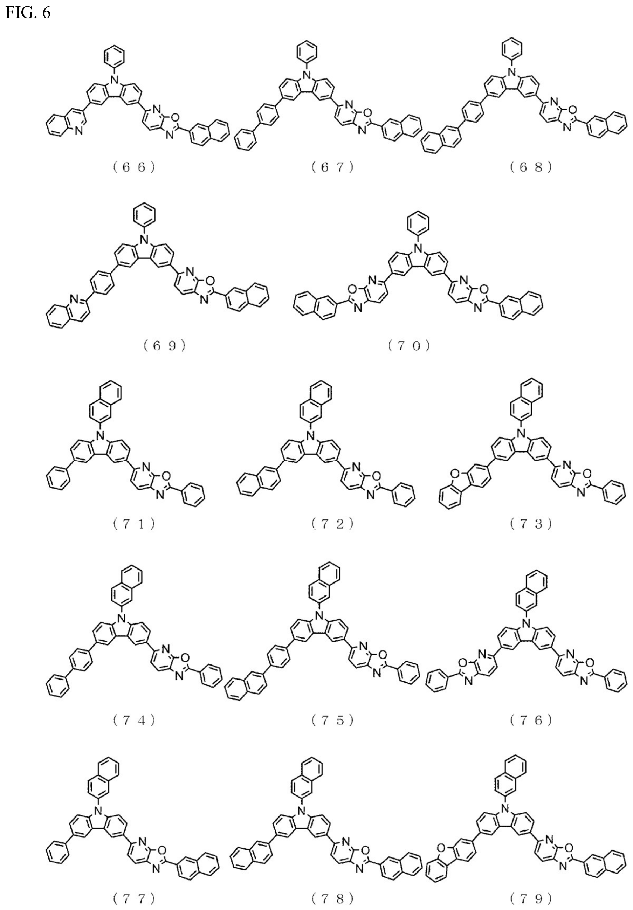

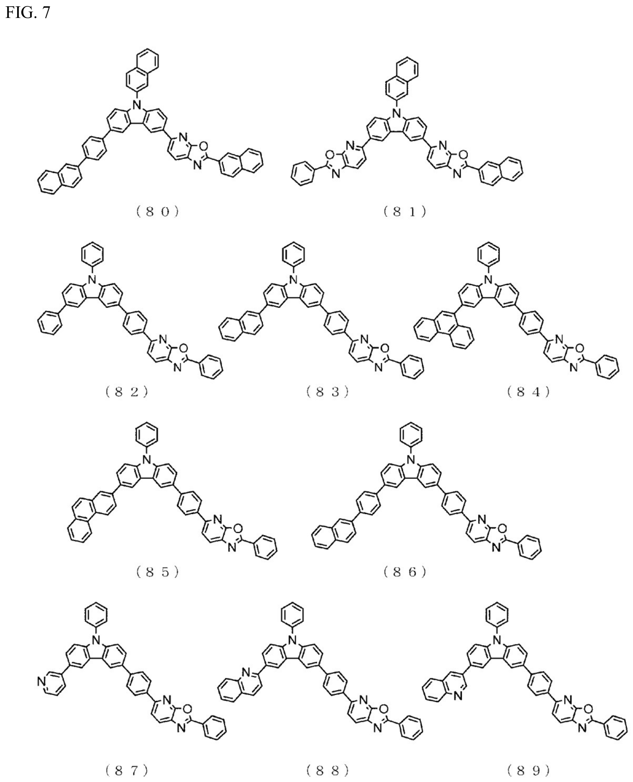

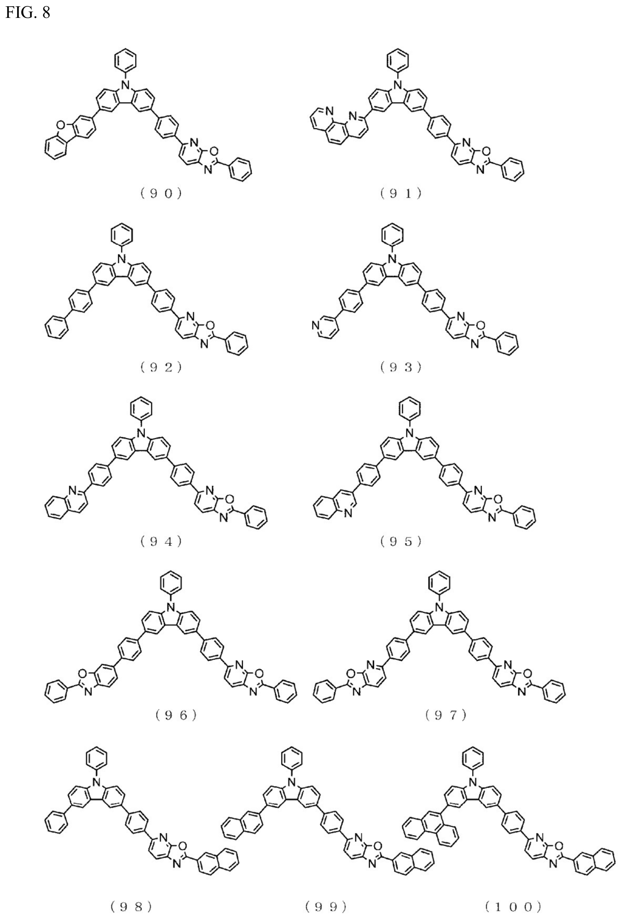

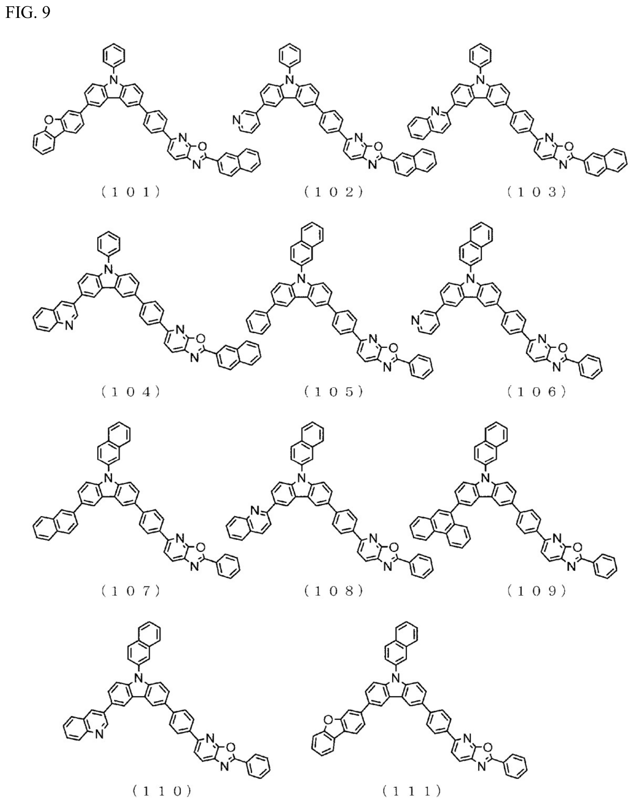

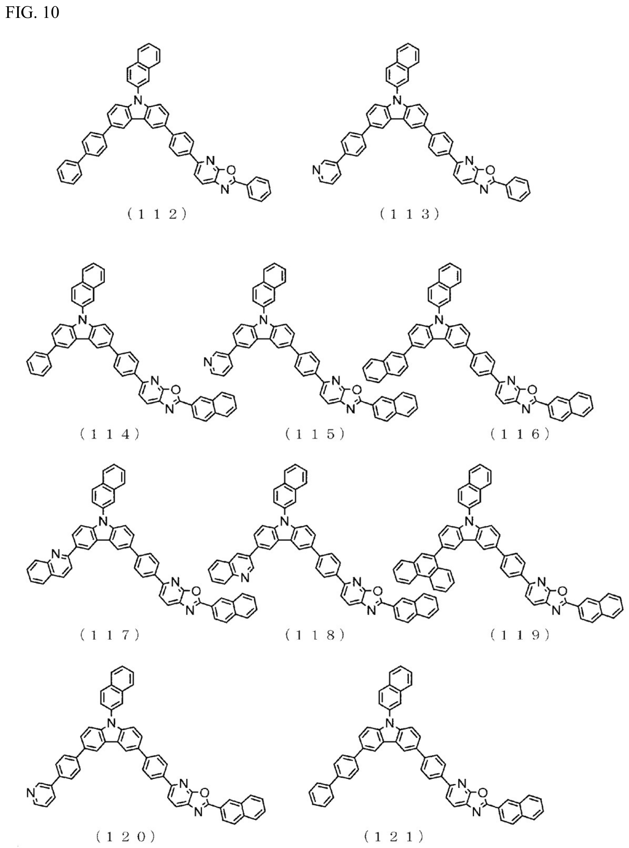

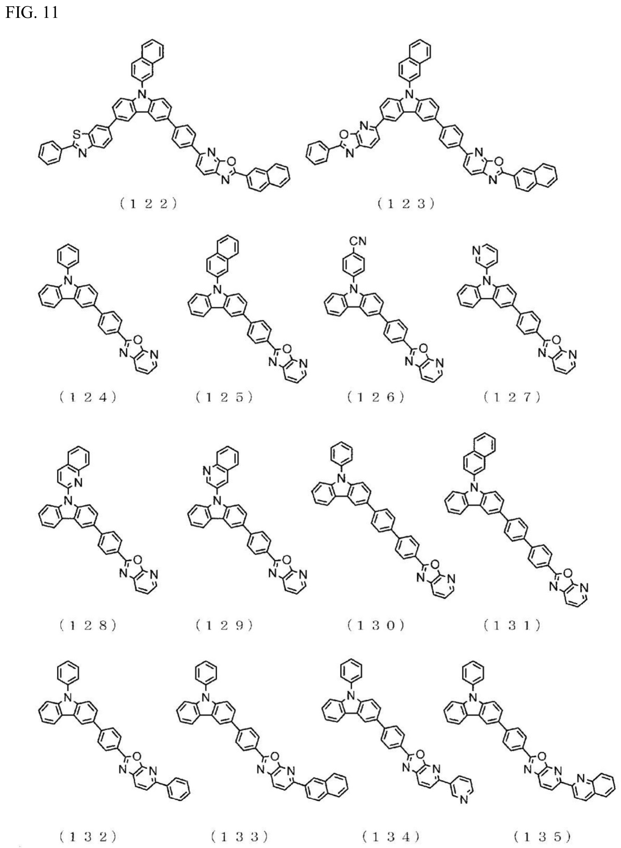

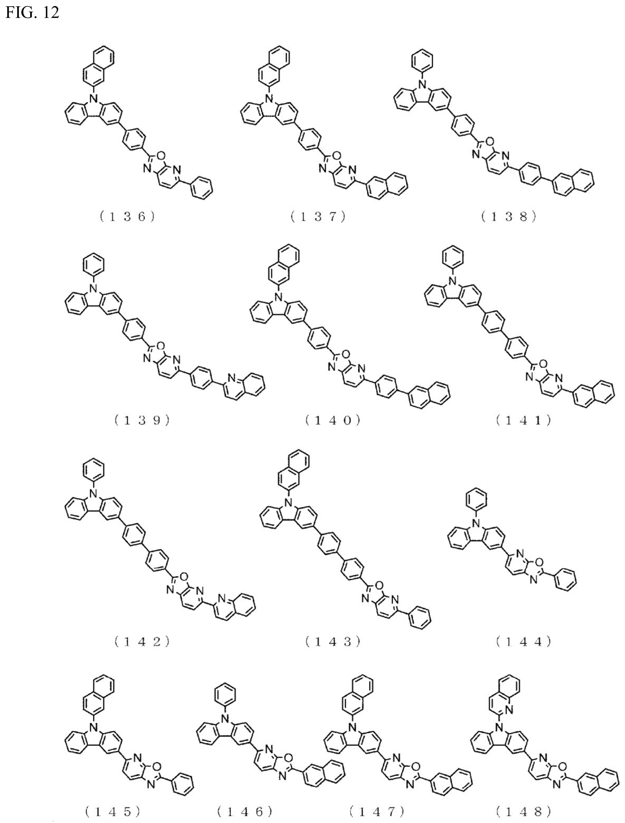

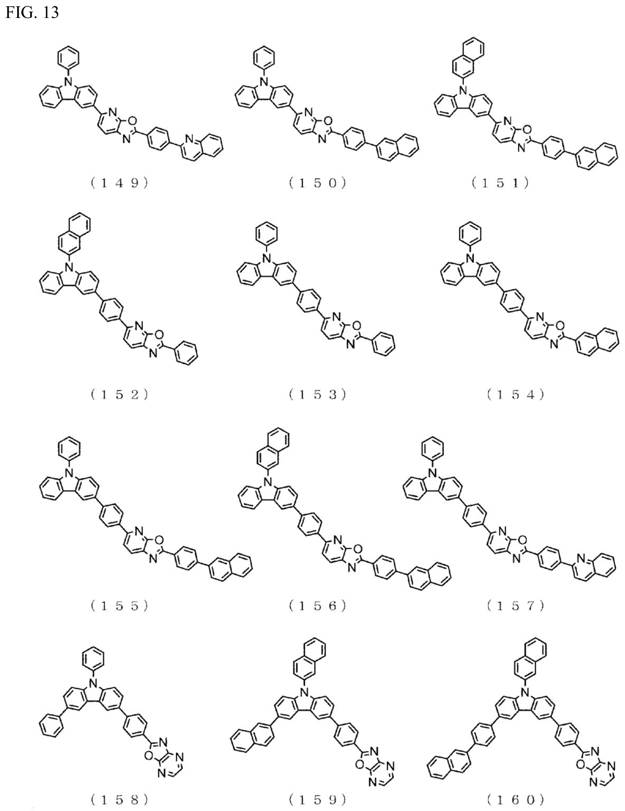

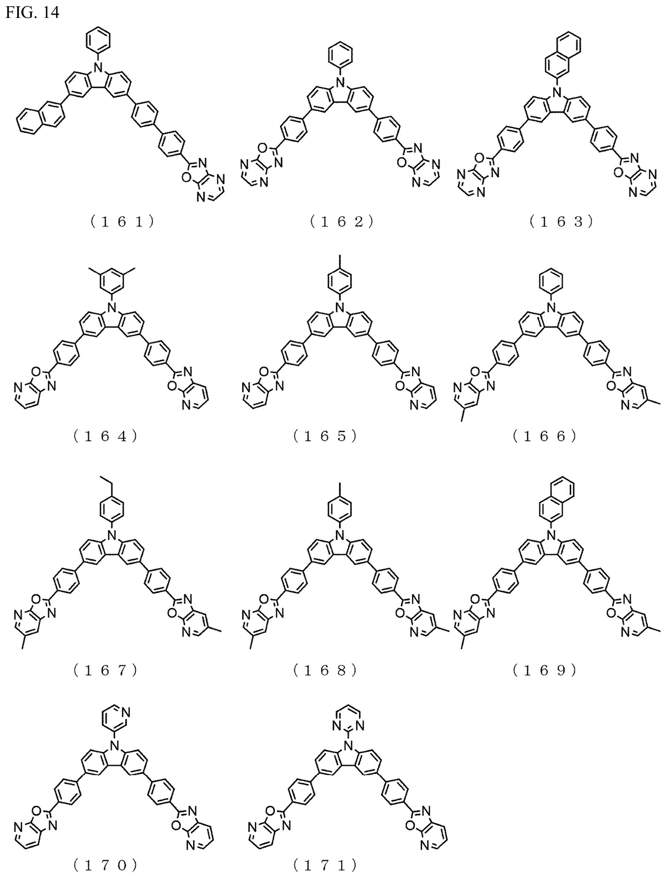

Among the carbazole compounds of the present invention, specific examples of preferable compounds are shown in Figures 1 to 14, but are not limited to these compounds.

Purification of the carbazole compounds of the present invention is not particularly limited, and can be carried out by known methods used for the purification of organic compounds, such as a purification method by column chromatography, an adsorption purification method by silica gel, activated carbon, activated clay, etc., a recrystallization method by a solvent or a standard purification method, a sublimation purification method, etc. Identification of compounds can be performed by NMR analysis.

For the physical property values of the carbazole compounds of the present invention, it is preferable to measure the melting point, glass transition temperature (Tg), refractive index, and extinction coefficient. The melting point is an indicator of deposition properties, the glass transition temperature (Tg) is an indicator of the stability in the thin film state, and the refractive index and extinction coefficient are indicators of improved light extraction efficiency.

The melting point and the glass transition temperature (Tg) can be measured with a high-sensitivity differential scanning calorimeter (DSC3100SA, manufactured by Bruker AXS) using powder.

The refractive index and extinction coefficient can be measured using a spectrometer (F10-RT-UV, manufactured by Philmetrics) on an 80 nm thin film formed on a silicon substrate.

The structure of the organic EL element may be for example, if the element is a light-emitting element of a top-emission structure, a structure in which an anode, a hole transport layer, a light-emitting layer, an electron transport layer, a cathode, and a capping layer are sequentially formed on a glass substrate, a structure in which a hole injection layer is formed between an anode and a hole transport layer, a structure in which an electron blocking layer is formed between the hole transport layer and the light-emitting layer, a structure in which a hole blocking layer is formed between the light-emitting layer and the electron transport layer, and a structure in which an electron injection layer is formed between an electron transport layer and a cathode. In these multilayer structures, it is possible for one organic layer to play the role of several layers, and for example, a feature of serving as both a hole injection layer and a hole transport layer, a feature of serving as both a hole transport layer and an electron blocking layer, a feature of serving as both a hole blocking layer and an electron transport layer, or a feature of serving as both an electron transport layer and an electron injection layer is also possible. In addition, it is possible to have a configuration in which two or more organic layers with the same function are stacked, a configuration in which two layers of hole transport layers are stacked, a configuration in which two layers of light-emitting layers are stacked, a configuration in which two layers of electron transport layers are stacked, and a configuration in which two layers of capping layers are stacked may be used.

The total film thickness of each layer of the organic EL element is preferably 200 nm to 750 nm, and more preferably 350 nm to 600 nm. In addition, the film thickness of the capping layer is, for example, preferably 30 nm to 120 nm, and more preferably 40 nm to 80 nm. In this case, excellent light extraction efficiency is achieved. The film thickness of the capping layer can be appropriately varied depending on the type of light-emitting material used in the light-emitting element, the thickness of the organic EL element other than the capping layer, and other factors.

For the anode of the organic EL element, an electrode material with a high work function, such as ITO or gold is used.

As a material for the hole injection layer of the organic EL element, an arylamine compound having three or more triphenylamine structures in the molecule, linked by a single bond or a divalent group that does not contain heteroatom such as a starburst type triphenylamine derivative, various triphenylamine tetramers, a porphyrin compound represented by copper phthalocyanine, acceptor-type heterocyclic compounds such as hexacyanoazatriphenylene, and coating-type polymer materials may be used. A single material can be used to form a film or different materials can be mixed together to form a film and used as one layer. In addition, they may be formed into a laminated structure by laminating only layers made of a single material, only layers made by mixing different materials, or layers made of a single material and layers made by mixing different materials. These materials can be formed into thin films by known methods such as vapor deposition, spin coating, and inkjet methods.

As a material for the hole transport layer of the organic EL element, benzidine derivatives such as N,N'-diphenyl-N,N'-di(m-tolyl)-benzidine (hereinafter, TPD), N,N'-diphenyl-N,N'-di(α-naphthyl)-benzidine, and N,N,N',N'-tetrabiphenylylbenzidine, and 1,1-bis[4-(di-4-tolylamino)phenyl]cyclohexane, etc. may be used. In particular, it is preferable to use an arylamine compound having two triphenylamine structures in the molecule, linked by a single bond or a divalent group that does not contain heteroatom, such as N,N,N',N'-tetrabiphenylylbenzidine. In addition, it is preferable to use an arylamine compound having three or more triphenylamine structures in the molecule, linked by a single bond or a divalent group that does not contain heteroatom, such as various triphenylamine trimers and tetramers. A single material can be used to form a film or different materials can be mixed together to form a film and used as one layer. In addition, they may be formed into a laminated structure by laminating only layers made of a single material, only layers made by mixing different materials, or layers made of a single material and layers made by mixing different materials. Further, as a material for the hole injection/transport layer, a coating type polymer material such as poly(3,4-ethylenedioxythiophene)/poly(styrenesulfonate) may be used. These materials can be formed into thin films by known methods such as vapor deposition, spin coating, and inkjet methods.

In addition, as a material for the hole injection layer or the hole transport layer, materials typically used in these layers can be P-doped with dopants such as trisbromophenylamine hexachloroantimony and radialene derivatives (see e.g., Patent Document 3), etc., as well as polymer compounds having a partial structure of a benzidine derivative such as TPD may be used.

It is also possible to laminate an electron blocking layer in an organic EL element. As a material for the electron blocking layer of the organic EL element, compounds having an electron blocking function, such as carbazole derivatives such as 4,4',4"-tri(N-carbazolyl)triphenylamine (hereinafter "TCTA"), 9,9-bis[4-(carbazol-9-yl)phenyl]fluorene, 1,3-bis(carbazol-9-yl)benzene (hereinafter "mCP"), and 2,2-bis(4-carbazol-9-yl-phenyl)adamantane, and compounds having a triphenylsilyl group and a triarylamine structure represented by 9-[4-(carbazole)-9-yl)phenyl]-9-[4-(triphenylsilyl)phenyl]-9H-fluorene, may be used. A single material can be used to form a film or different materials can be mixed together to form a film and used as one layer. In addition, they may be formed into a laminated structure by laminating only layers made of a single material, only layers made by mixing different materials, or layers made of a single material and layers made by mixing different materials. These materials can be formed into thin films by known methods such as vapor deposition, spin coating, and inkjet methods.

As a material for the light-emitting layer of the organic EL element, metal complexes of quinolinol derivatives including Alq₃, and various metal complexes, anthracene derivatives, bis-styrylbenzene derivatives, pyrene derivatives, oxazole derivatives, polyparaphenylenevinylene derivatives, etc. may be used. In addition, the light-emitting layer may be composed of a host material and a dopant material, and an anthracene derivative is preferably used as the host material. However, in addition to the above light-emitting materials, heterocyclic compounds having an indole ring as a partial structure of a condensed ring, heterocyclic compounds having a carbazole ring as a partial structure of a condensed ring, carbazole derivatives, thiazole derivatives, benzimidazole derivatives, polydialkylfluorene derivatives, etc. may be used. Further, quinacridone, coumarin, rubrene, perylene and their derivatives, benzopyran derivatives, rhodamine derivatives, aminostyryl derivatives, etc. may be used as the dopant material, and it is particularly preferable to use a green light-emitting material. A single material can be used to form a film or different materials can be mixed together to form a film and used as one layer. In addition, they may be formed into a laminated structure by laminating only layers made of a single material, only layers made by mixing different materials, or layers made of a single material and layers made by mixing different materials.

In addition, it is also possible to use a phosphorescent emitter as a light-emitting material. A phosphorescent emitter of a metal complex such as iridium or platinum complex may be used as the phosphorescent emitter. For example, a green phosphorescent emitter such as Ir(ppy)₃, a blue phosphorescent emitter such as FIrpic or FIr₆, a red phosphorescent emitter such as Btp₂Ir(acac), etc., may be used and it is particularly preferable to use a green phosphorescent emitter. For the host material at this time, carbazole derivatives such as 4,4'-di(N-carbazolyl)biphenyl , TCTA, and mCP may be used as a host material with hole injection/transport properties, and p-bis(triphenylsilyl)benzene, 2,2',2"-(1,3,5-phenylene)-tris(1-phenyl-1H-benzimidazole), or the like may be used as a host material with electron transport properties.

In order to avoid concentration quenching, doping of the host material of the phosphorescent light-emitting material is preferably carried out by co-deposition in the range of 1 to 30 weight percent with respect to the entire light-emitting layer.

In addition, as the light-emitting material, it is also possible to use a material that emits delayed fluorescence, such as CDCB derivatives such as PIC-TRZ, CC2TA, PXZ-TRZ, and 4CzIPN (see e.g., Non-Patent Document 5). These materials may be formed into thin films by known methods such as vapor deposition, spin coating, and inkjet methods.

Further, a hole blocking layer can be laminated in an organic EL element. As a material for the hole blocking layer, compounds having a hole blocking function, such as phenanthroline derivatives such as basocuproine, metal complexes of quinolinol derivatives such as aluminum(III)bis(2-methyl-8-quinolinate)-4-phenylphenolate (hereinafter "BAlq"), various rare earth complexes, triazole derivatives, triazine derivatives, pyrimidine derivatives, oxadiazole derivatives, and benzoazole derivatives, may be used. These materials may also be used as materials for the electron transport layer. A single material can be used to form a film or different materials can be mixed together to form a film and used as one layer. In addition, they may be formed into a laminated structure by laminating only layers made of a single material, only layers made by mixing different materials, or layers made of a single material and layers made by mixing different materials. These materials can be formed into thin films by known methods such as vapor deposition, spin coating, and inkjet methods.

As a material for the electron transport layer of the organic EL element, metal complexes of quinolinol derivatives including Alq₃ and BAlq, various metal complexes, triazole derivatives, triazine derivatives, pyrimidine derivatives, oxadiazole derivatives, pyridine derivatives, benzimidazole derivatives, benzoazole derivatives, thiadiazole derivatives, anthracene derivatives, carbodiimide derivatives, quinoxaline derivatives, pyridoindole derivatives, phenanthroline derivatives, and silole derivatives, etc. may be used. A single material can be used to form a film or different materials can be mixed together to form a film and used as one layer. In addition, they may be formed into a laminated structure by laminating only layers made of a single material, only layers made by mixing different materials, or layers made of a single material and layers made by mixing different materials. These materials can be formed into thin films by known methods such as vapor deposition, spin coating, and inkjet methods.

As a material for the electron injection layer of the organic EL element, alkali metal salts such as lithium fluoride and cesium fluoride, alkaline earth metal salts such as magnesium fluoride, metal complexes of quinolinol derivatives such as lithium quinolinol, metal oxides such as aluminum oxide, and metals such as ytterbium (Yb), samarium (Sm), calcium (Ca), strontium (Sr), and cesium (Cs) may be used. The electron injection layer may be omitted depending on the preferable selection of the electron transport layer and cathode.

In addition, for the electron injection layer and the electron transport layer, materials typically used in these layers can be N-doped with metals such as cesium.

As a material for the cathode of the organic EL element, a metal with a low work function such as aluminum, an alloy with a lower work function, such as a magnesium silver alloy, a magnesium calcium alloy, a magnesium indium alloy, and an aluminum magnesium alloy, as well as ITO and IZO, are used.

As a capping layer of the organic EL element, the carbazole compounds of the present invention are preferably used. A single material can be used to form a film or different materials can be mixed together to form a film and used as one layer. In addition, they may be formed into a laminated structure by laminating only layers made of a single material, only layers made by mixing different materials, or layers made of a single material and layers made by mixing different materials. These materials can be formed into thin films by known methods such as vapor deposition, spin coating, and inkjet methods.

When the carbazole compound of the present invention is formed into a film, the refractive index of the film is preferably 1.70 or greater and particularly preferably 1.85 or greater when the wavelength of light passing through the film is in the range of 450 nm to 700 nm.

In addition, although an organic element of a top-emission structure has been described above, the present invention is not limited thereto. The same applies to an organic EL element of a bottom-emission structure, or an organic EL element of a dual emission structure in which light is emitted from both the top and bottom portions. In these cases, the electrodes positioned in the direction in which light is emitted from the light-emitting element to the outside are preferably transparent or translucent.

### [MODE FOR CARRYING OUT THE INVENTION]

### Examples

Hereinafter, embodiments of the present invention will be described in detail by way of Examples. However, as long as it does not go beyond the core of the invention, the present invention is not limited to the following Examples.

### [Example 1]

### <Synthesis of 3,6-bis{4-(oxazolo[5,4-b]pyridin-2-yl)phenyl}-9-phenyl-9H-carbazole (Compound (33))>

Into a reaction vessel, 10.5 g of 3,6-dibromo-9-phenyl-9H-carbazole,
17.7 g of 2-{4-(4,4,5,5-tetramethyl-1,3,2-dioxaborolan-2-yl)phenyl}oxazolo[5,4-b]pyridine,
0.6 g of Tetrakis(triphenylphosphine)palladium (0), and 10.9 g of potassium carbonate were injected, and the mixture was refluxed and stirred under a toluene/EtOH/H₂O mixed solvent overnight. After cooling, MeOH was added, and the precipitated solid was filtered to obtain a crude product. The crude product was subjected to purification by recrystallization using a monochlorobenzene solvent to obtain 7.2 g (yield: 43.5%) of 3,6-bis{4-(oxazolo[5,4-b]pyridin-2-yl)phenyl}-9-phenyl-9H-carbazole (Compound 33) as a pale yellow powder.

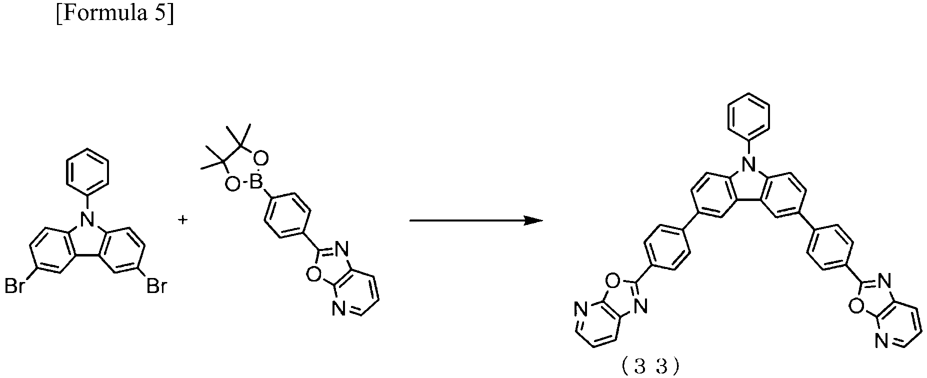

The structure of the obtained pale yellow powder was identified using NMR.

The following 25 hydrogen signals were detected by ¹H-NMR (CDCl₃), confirming that it was Compound 33.
δ (ppm) =8. 55 (2H) , 8. 42 (4H) , 8. 38 (2H) , 8. 10 (2H) , 7. 96 (4H), 7. 79 (2H) , 7. 68 (2H) , 7. 66 (2H), 7. 54 ( 3H) , 7. 38 (2H) _{∘}

### [Example 2]

<Synthesis of 3,6-bis{4-(oxazolo[5,4-b]pyridin-2-yl)phenyl}-9-(naphthalen-2-yl)-9H-carbazole (Compound (47))>

Into a reaction vessel, 10.0 g of 3,6-dibromo-9-(naphthalen-2-yl)-9H-carbazole,
15.7 g of 2-{4-(4,4,5,5-tetramethyl-1,3,2-dioxaborolan-2-yl)phenyl}oxazolo[5,4-b]pyridine,
0.8 g of Tetrakis(triphenylphosphine)palladium (0), and 9.2 g of potassium carbonate were injected and the mixture was refluxed and stirred under a toluene/EtOH/H₂O mixed solvent overnight. After cooling, MeOH was added, and the precipitated solid was filtered to obtain a crude product. The crude product was subjected to purification by recrystallization using a monochlorobenzene solvent to obtain 8.3 g (yield: 54.9%) of 3,6-bis{4-(oxazolo[5,4-b]pyridin-2-yl)phenyl}-9-(naphthalen-2-yl)-9H-carbazole (Compound 47) as a pale yellow powder.

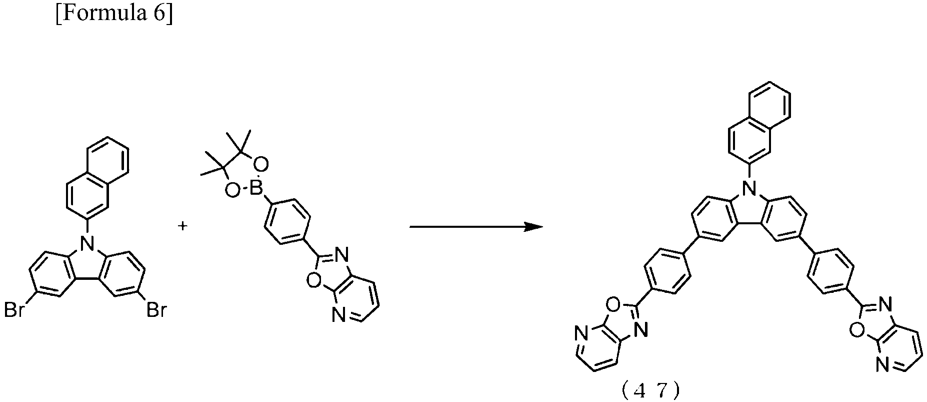

The structure of the obtained pale yellow powder was identified using NMR.

The following 27 hydrogen signals were detected by ¹H-NMR (CDCl₃), confirming that it was Compound 47.
δ (ppm) =8. 57 (2H) , 8. 42 (4H) , 8. 3 7 (2H) , 8. 14 (1 H) , 8. 12 (1H) , 8. 10 (2H) , 8. 02 (1H), 7. 96 (5H) , 7 . 79 (2H) , 7. 73 (1H) , 7. 64 (2H) , 7. 58 (2H) , 7. 38 ( 2 H) _{∘}

### [Example 3]

### <Synthesis of 3,6-bis{4-(oxazolo[5,4-b]pyridin-2-yl)phenyl}-9-(3,5-dimethylphenyl)-9H-carbazole (Compound 164)>

To a nitrogen-purged reaction vessel, 8.0 g of 3,6-dibromo-9-(3,5-dimethylphenyl)-9H-carbazole, 13.2 g of 2-(4-(4,4,5,5-tetramethyl-1,3,2-dioxaborolan-2-yl)phenyl)oxazolo[5,4-b]pyridine, 7.7 g of potassium carbonate, 0.9 g of Tetrakis(triphenylphosphine)palladium (0), 65 ml of toluene, 40 ml of ethanol, and 26 ml of municipal water were added, and the mixture was stirred overnight under heated reflux.

After checking the completion of the reaction, methanol was added, and the resulting precipitate was filtered to obtain a crude product. The resulting solid was dissolved in heated monochlorobenzene, then silica gel was added thereto and stirred, and filtration through Celite was carried out. The filtrate was concentrated to obtain a crude product, which was subjected to standard purification using a monochlorobenzene solvent to obtain 6.5 g (yield: 52.8%) of 3,6-bis{4-(oxazolo[5,4-b]pyridin-2-yl)phenyl}-9-(3,5-dimethylphenyl)-9H-carbazole (Compound 164).

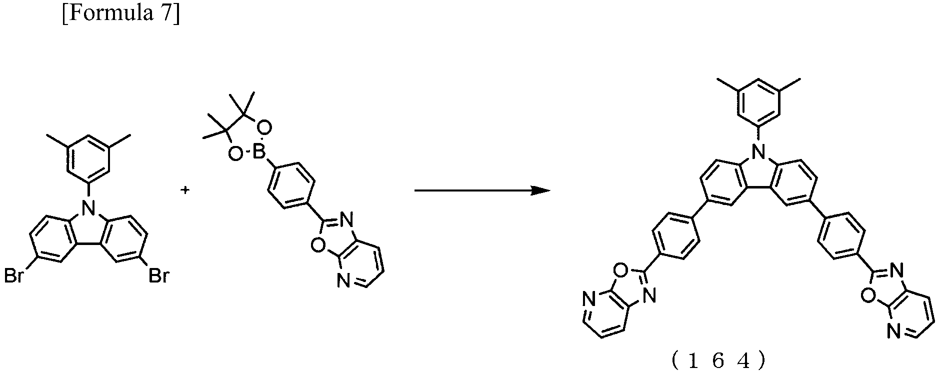

The structure of the obtained pale yellow powder was identified using NMR.

The following 31 hydrogen signals were detected by ¹H-NMR (CDCl₃).

δ (ppm)=8.56 (2H), 8.44 (4H), 8.40 (2H), 8.13 (2H), 7.98 (4H), 7.80 (2H), 7.55 (2H), 7.42-7.39 (2H), 7.28-7.26 (4H), 7.20 (1H), 2.50 (6H).

### [Example 4]

### <Synthesis of 3,6-bis{4-(oxazolo[5,4-b]pyridin-2-yl)phenyl}-9-(4-methylphenyl)-9H-carbazole (Compound 165)>

To a nitrogen-purged reaction vessel, 8.0 g of 3,6-dibromo-9-(4-methylphenyl)-9H-carbazole, 13.7 g of 2-(4-(4,4,5,5-tetramethyl-1,3,2-dioxaborolan-2-yl)phenyl)oxazolo[5,4-b]pyridine, 8.0 g of potassium carbonate, 0.9 g of Tetrakis(triphenylphosphine)palladium (0), 70 ml of toluene, 42 ml of ethanol, and 28 ml of municipal water were added and stirred overnight under heated reflux.

After checking the completion of the reaction, methanol was added, and the resulting precipitate was filtered to obtain a crude product. The resulting solid was dissolved in heated monochlorobenzene, then silica gel was added thereto and stirred, and filtration through Celite was carried out. The filtrate was concentrated to obtain a crude product, which was subjected to standard purification using a monochlorobenzene/acetone mixed solvent to obtain 6.8 g (yield: 54.8%) of 3,6-bis{4-(oxazolo[5,4-b]pyridin-2-yl)phenyl}-9-(4-methylphenyl)-9H-carbazole (Compound 165).

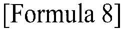

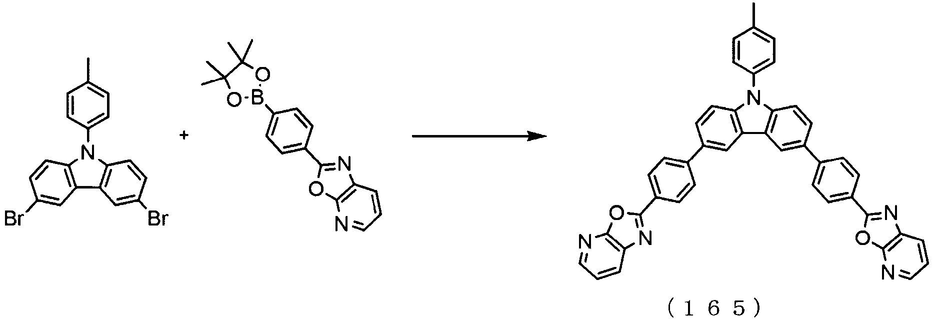

The structure of the obtained pale yellow powder was identified using NMR.

The following 27 hydrogen signals were detected by ¹H-NMR (CDCl₃).

δ (ppm)=8.54 (2H), 8.43 (4H), 8.39 (2H), 8.11 (2H), 7.96 (4H), 7.79 (2H), 7.53-7.47 (6H), 7.41-7.38 (2H), 2.55 (3H).

### [Example 5]

### <Synthesis of 3,6-bis{4-(6-methyloxazolo[5,4-b]pyridin-2-yl)phenyl}-9-phenyl-9H-carbazole (Compound 166)>

To a nitrogen-purged reaction vessel, 5.0 g of 3,6-dibromo-9-phenyl-9H-carbazole, 8.8 g of 6-methyl-2-(4-(4,4,5,5-tetramethyl-1,3,2-dioxaborolan-2-yl)phenyl)oxazolo[5,4-b]pyridine, 5.2 g of potassium carbonate, 0.6 g of Tetrakis(triphenylphosphine)palladium (0), 35 ml of toluene, 15 ml of ethanol, and 10 ml of municipal water were added and stirred overnight under heated reflux.

After checking the completion of the reaction, methanol and municipal water were added, and the resulting precipitate was filtered to obtain a solid. The resulting solid was dissolved in heated dichlorobenzene, then silica gel was added thereto and stirred, and filtration through Celite was carried out. The filtrate was concentrated to obtain a crude product, which was subjected to standard purification using a dichlorobenzene solvent to obtain 5.0 g (yield: 60.8%) of 3,6-bis{4-(6-methyloxazolo[5,4-b]pyridin-2-yl)phenyl}-9-phenyl-9H-carbazole (Compound 166).

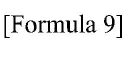

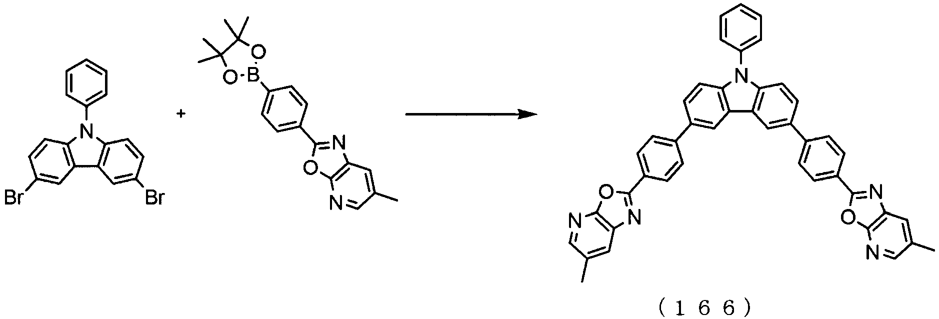

The structure of the obtained pale yellow powder was identified using NMR.

The following 29 hydrogen signals were detected by ¹H-NMR (CDCl₃).

δ (ppm)=8.55 (2H), 8.42 (4H), 8.21 (2H), 7.97-7.91 (6H), 7.80 (2H), 7.72-7.54 (7H), 2.53 (6H).

### [Example 6]

### <Synthesis of 3,6-bis{4-(6-methyloxazolo[5,4-b]pyridin-2-yl)phenyl}-9-(4-ethylphenyl)-9H-carbazole (Compound 167)>

To a nitrogen-purged reaction vessel, 7.0 g of 3,6-dibromo-9-(4-ethylphenyl)-9H-carbazole, 12.1 g of 6-methyl-2-(4-(4,4,5,5-tetramethyl-1,3,2-dioxaborolan-2-yl)phenyl)oxazolo[5,4-b]pyridine, 6.8 g of potassium carbonate, 0.8 g of Tetrakis(triphenylphosphine)palladium (0), 35 ml of toluene, 21 ml of ethanol, and 14 ml of municipal water were added, and the mixture was stirred overnight under heated reflux.

After checking the completion of the reaction, methanol and municipal water were added, and the resulting precipitate was filtered to obtain a solid. The resulting solid was dissolved in heated dichlorobenzene, then silica gel was added thereto and stirred, and filtration through Celite was carried out. The filtrate was concentrated to obtain a crude product, which was subjected to standard purification using a dichlorobenzene solvent to obtain 6.0 g (yield: 53.8%) of 3,6-bis{4-(6-methyloxazolo[5,4-b]pyridin-2-yl)phenyl}-9-(4-ethylphenyl)-9H-carbazole (Compound 167).

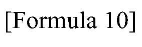

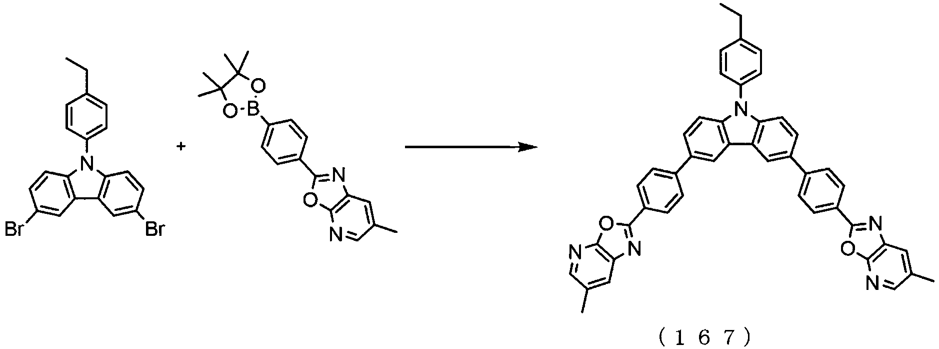

The structure of the obtained pale yellow powder was identified using NMR.

The following 33 hydrogen signals were detected by ¹H-NMR (CDCl₃).

δ (ppm)=8.55 (2H), 8.42 (4H), 8.21 (2H), 7.96 (4H), 7.92 (2H), 7.80 (2H), 7.57-7.50 (6H), 2.86 (2H), 2.54 (6H), 1.40 (3 H).

### [Example 7]

### <Synthesis of 3,6-bis{4-(6-methyloxazolo[5,4-b]pyridin-2-yl)phenyl}-9-(4-methylphenyl)-9H-carbazole (Compound 168)>

To a nitrogen-purged reaction vessel, 8.0 g of 3,6-dibromo-9-(4-methylphenyl)-9H-carbazole, 13.6 g of 6-methyl-2-(4-(4,4,5,5-tetramethyl-1,3,2-dioxaborolan-2-yl)phenyl)oxazolo[5,4-b]pyridine, 8.0 g of potassium carbonate, 0.7 g of Tetrakis(triphenylphosphine)palladium (0), 56 ml of toluene, 24 ml of ethanol, and 16 ml of municipal water were added, and the mixture was stirred overnight under heated reflux.

After checking the completion of the reaction, methanol and municipal water were added, and the resulting precipitate was filtered to obtain a solid. The resulting solid was dissolved in heated dichlorobenzene, then silica gel was added thereto and stirred, and filtration through Celite was carried out. The filtrate was concentrated to obtain a crude product, which was subjected to standard purification using a dichlorobenzene solvent to obtain 7.8 g (yield: 60.1%) of 3,6-bis{4-(6-methyloxazolo[5,4-b]pyridin-2-yl)phenyl}-9-(4-methylphenyl)-9H-carbazole (Compound 168).

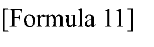

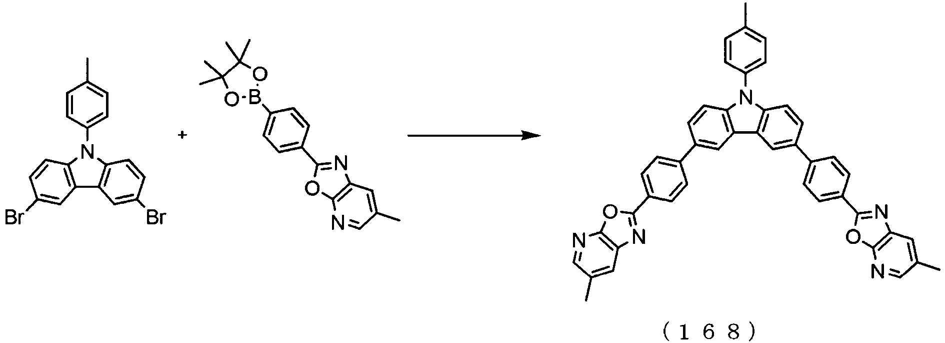

The structure of the obtained pale yellow powder was identified using NMR.

The following 31 hydrogen signals were detected by ¹H-NMR (CDCl₃).

δ (ppm)=8.57 (2H), 8.42 (4H), 8.22 (2H), 7.98-7.92 (6H), 7.80 (2H), 7.54-7.42 (6H), 2.56 (3H), 2.54 (6H).

### [Example 8]

### <Synthesis of 3,6-bis {4-(6-methyloxazolo[5,4-b]pyridin-2-yl)phenyl}-9-(naphthalen-2-yl)-9H-carbazole (Compound 169)>

To a nitrogen-purged reaction vessel, 7.5 g of 3,6-dibromo-9-(naphthalen-2-yl)-9H-carbazole, 12.3 g of 6-methyl-2-(4-(4,4,5,5-tetramethyl-1,3,2-dioxaborolan-2-yl)phenyl)oxazolo[5,4-b]pyridine, 6.9 g of potassium carbonate, 0.8 g of Tetrakis(triphenylphosphine)palladium (0), 38 ml of toluene, 23 ml of ethanol, and 15 ml of municipal water were added, and the mixture was stirred overnight under heated reflux.

After checking the completion of the reaction, methanol was added, and the resulting precipitate was filtered to obtain a solid. The resulting solid was dissolved in heated dichlorobenzene, then silica gel was added thereto and stirred, and filtration through Celite was carried out. The filtrate was concentrated to obtain a crude product, which was subjected to standard purification using a dichlorobenzene solvent to obtain 7.2 g (yield: 61.4%) of 3,6-bis{4-(6-methyloxazolo[5,4-b]pyridin-2-yl)phenyl}-9-(naphthalen-2-yl)-9H-carbazole (Compound 169).

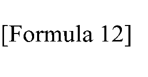

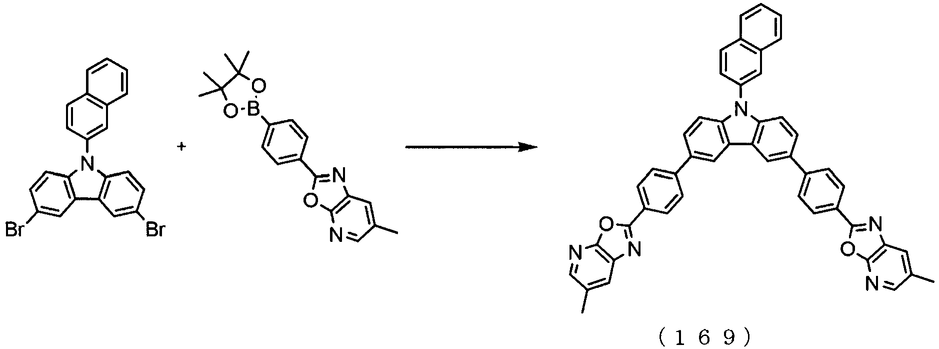

The structure of the obtained pale yellow powder was identified using NMR.

The following 31 hydrogen signals were detected by ¹H-NMR (CDCl₃).

δ (ppm)=8.56 (2H), 8.43 (4H), 8.22-8.14 (4H), 8.05 (1H), 7.98 (5H), 7.92 (2H), 7.82 (2H), 7.75 (1H), 7.66 (2H), 7.61 (2H), 2.54 (6H).

### [Example 9]

### <Synthesis of 3,6-bis{4-(oxazolo[5,4-b]pyridin-2-yl)phenyl}-9-(pyridin-3-yl)-9H-carbazole (Compound 170)>

To a nitrogen-purged reaction vessel, 14.0 g of 3,6-bis(4,4,5,5-tetramethyl-1,3,2-dioxaborolan-2-yl)-9-(pyridin-3-yl)-9H-carbazole, 16.3 g of 2-(4-bromophenyl)oxazolo[5,4-b]pyridine, 19.5 g of potassium carbonate, 3.3 g of Tetrakis(triphenylphosphine)palladium (0), 150 ml of toluene, 100 ml of ethanol, and 150 ml of municipal water were added, and the mixture was stirred overnight under heated reflux.

After checking the completion of the reaction, the resulting precipitate was filtered to obtain a solid. The resulting solid was dissolved in heated monochlorobenzene, then silica gel was added thereto and stirred, and filtration through Celite was carried out. The filtrate was concentrated to obtain a crude product, which was subjected to standard purification using a monochlorobenzene solvent to obtain 2.2 g (yield: 12.4%) of 3,6-bis{4-(oxazolo[5,4-b]pyridin-2-yl)phenyl}-9-(pyridin-3-yl)-9H-carbazole (Compound 170).

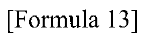

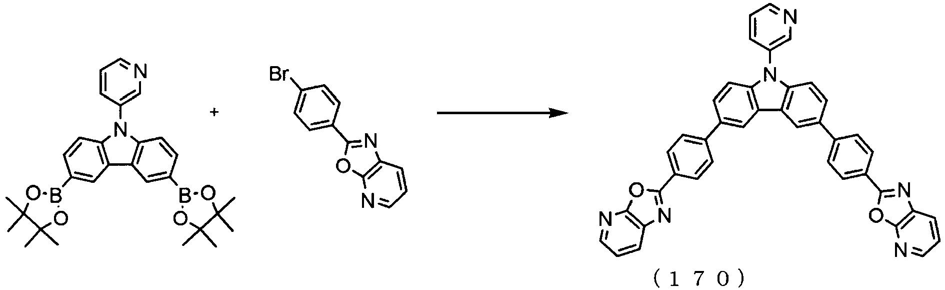

The structure of the obtained pale yellow powder was identified using NMR.

The following 24 hydrogen signals were detected by ¹H-NMR (CDCl₃).

δ (ppm)=9.02 (1H), 8.84 (1H), 8.56 (2H), 8.44 (4H), 8.40 (2H), 8.12 (3H), 7.97 (4H), 7.81 (2H), 7.73 (1H), 7.54 (2H), 7.42-7.38 (2H).

### [Example 10]

For the compounds obtained in Examples 1 to 9, the melting points and glass transition temperatures (Tg) were measured using a high-sensitivity differential scanning calorimeter (DSC3100SA manufactured by Bruker AXS). The results are shown in Table 1.

**[Table 1]**

| | Melting point | Glass transition temperature |
|---|---|---|
| Compound (33) | 346.0 | - |
| Compound (47) | 338.0 | 162.5 |
| Compound (164) | 358.5 | 156.6 |
| Compound (165) | 334.6 | 154.0 |
| Compound (166) | 369.9 | - |
| Compound (167) | 382.6 | - |
| Compound (168) | 401.1 | - |
| Compound (169) | 366.6 | 273 |
| Compound (170) | 345.1 | - |

From the results shown above, it can be seen that the carbazole compounds of the present invention obtained in Examples 1 to 9 have high melting points and either have no glass transition temperature or have glass transition temperatures of 100°C or higher. This indicates that the thin film states are stable.

### [Example 11]

Vapor-deposited films with a film thickness of 80 nm were prepared on a silicon substrate using the compounds obtained from Examples 1 to 9, and refractive index n and extinction coefficient k were measured at wavelengths of 450 nm and 750 nm by a spectrometer (F10-RT-UV manufactured by Filmetrics). Further, for comparison, measurements were also performed for Alq₃ and a comparative compound (CPL-1) having the following structural formula (see, e.g., Patent Document 4). The measurement results are summarized in Table 2.

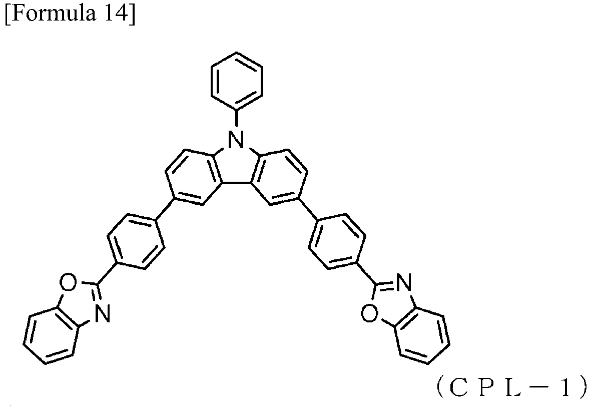

**[Table 2]**

| | Refractive index n (λ:450nm) | Refractive index n (λ:750nm) | Extinction coefficient (λ: 450nm) | Extinction coefficient (λ: 750nm) |
|---|---|---|---|---|
| Compound (33) | 2.34 | 1.97 | 0.00 | 0.00 |
| Compound (47) | 2.36 | 1.97 | 0.00 | 0.00 |
| Compound (164) | 2.25 | 1.92 | 0.00 | 0.00 |
| Compound (165) | 2.32 | 1.95 | 0.00 | 0.00 |
| Compound (166) | 2.30 | 1.94 | 0.01 | 0.00 |
| Compound (167) | 2.23 | 1.90 | 0.00 | 0.00 |
| Compound (168) | 2.25 | 1.93 | 0.00 | 0.00 |
| Compound (169) | 2.29 | 1.95 | 0.00 | 0.00 |
| Compound (170) | 2.34 | 1.97 | 0.00 | 0.00 |
| Alq₃ | 1.88 | 1.73 | 0.03 | 0.00 |
| Compound (CPL-1) | 2.19 | 1.92 | 0.00 | 0.00 |

As shown in Table 2, the carbazole compounds of the present invention in the wavelength range of 450 nm to 750 nm have extinction coefficients and refractive indexes equal to or greater than those of Alq₃ and the comparative compound (CPL-1). By using the carbazole compounds of the present invention as a material for the capping layer, improvement in light extraction efficiency in organic EL elements can be expected.

### [Example 12]

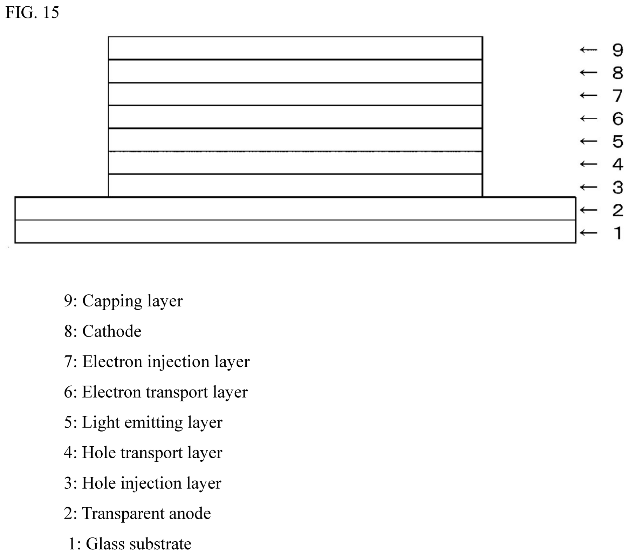

As an example shown in FIG. 15, the organic EL element of the present invention was manufactured by depositing a hole injection layer 3, a hole transport layer 4, a light-emitting layer 5, an electron transport layer 6, an electron injection layer 7, a cathode 8, and a capping layer 9 in that order on a glass substrate 1 on which a reflective ITO electrode had previously been formed as a transparent anode 2.

Specifically, after a glass substrate 1 on which ITO with a film thickness of 50 nm, a silver alloy reflective film with a film thickness of 100 nm, and ITO with a film thickness of 5 nm were sequentially formed was ultrasonic cleaned in isopropyl alcohol for 20 minutes, drying was performed for 10 minutes on a hot plate heated to 250°C. After that, UV ozone treatment was performed for 2 minutes, and then this glass substrate on which ITO was formed was mounted in a vacuum deposition equipment, and the pressure was reduced to 0.001 Pa or less. Subsequently, an electron acceptor (Acceptor-1) of the following structural formula and Compound (HTM-1) of the following structural formula as a hole injection layer 3 to cover the transparent anode 2 were subjected to binary deposition at a deposition rate in which a deposition rate ratio becomes Acceptor-1:Compound (HTM-1) = 3:97, and thus formed to have a film thickness of 10 nm.

On this hole injection layer 3, Compound (HTM-1) of the following structural formula was formed as a hole transport layer 4 to have a film thickness of 140 nm. On this hole transport layer 4, Compound (EMD-1) of the following structural formula and Compound (EMH-1) of the following structural formula as the light-emitting layer 5 were subjected to binary deposition at a deposition rate in which a deposition rate ratio becomes EMD-1:EMH-1 = 5:95, and thus formed to have a film thickness of 20 nm. On this light-emitting layer 5, Compound (ETM-1) having the following structural formula and Compound (ETM-2) having the following structural formula as the electron transport layer 6 were subjected to binary deposition at a deposition rate in which a deposition rate ratio becomes ETM-1:ETM-2 = 50:50, and thus formed to have a film thickness of 30 nm. On this electron transport layer 6, lithium fluoride was formed as the electron injection layer 7 to have a film thickness of 1 nm. On this electron injection layer 7, a magnesium silver alloy was formed as the cathode 8 to have a film thickness of 12 nm.

Finally, Compound (33) of Example 1 was formed as the capping layer 9 to have a film thickness of 60 nm. The properties of the manufactured organic EL element were measured in the air and at room temperature and Table 3 summarizes and shows the measurement results of the luminescence characteristics when a direct current voltage is applied to the manufactured organic EL element.

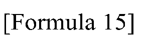

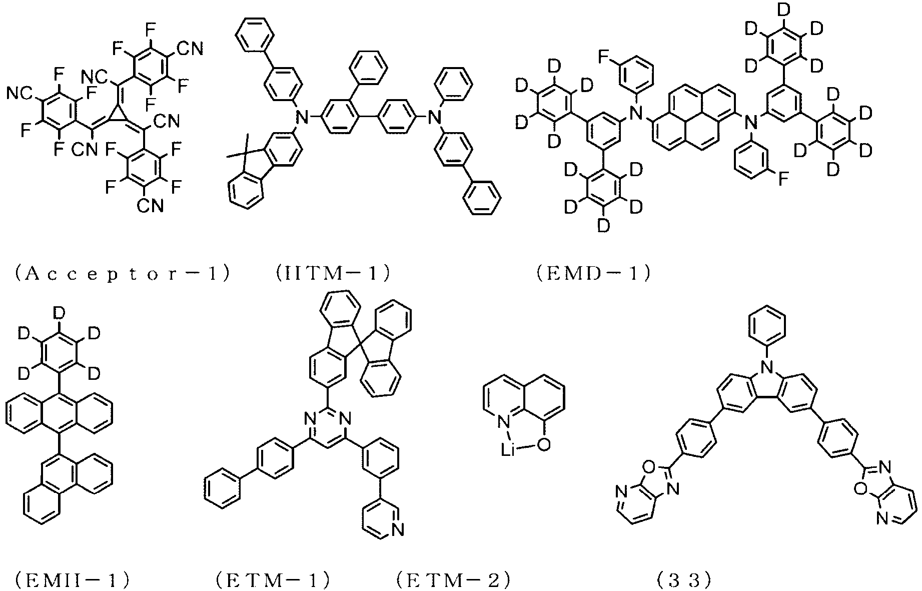

### [Example 13]

Organic EL elements were manufactured under the same conditions except that the Compound (47) obtained in Example 2 was used instead of Compound (33) in Example 1 as the capping layer 9 in Example 12. The properties of the manufactured organic EL elements were measured in the air and at room temperature and Table 3 summarizes and shows the measurement results of the luminescence characteristics when a direct current voltage is applied to the manufactured organic EL elements.

### [Example 14]

Organic EL elements were manufactured under the same conditions except that the Compound (164) obtained in Example 3 was used instead of Compound (33) in Example 1 as the capping layer 9 in Example 12. The properties of the manufactured organic EL elements were measured in the air and at room temperature and Table 3 summarizes and shows the measurement results of the luminescence characteristics when a direct current voltage is applied to the manufactured organic EL elements.

### [Example 15]

Organic EL elements were manufactured under the same conditions except that the Compound (165) obtained in Example 4 was used instead of Compound (33) in Example 1 as the capping layer 9 in Example 12. The properties of the manufactured organic EL elements were measured in the air and at room temperature and Table 3 summarizes and shows the measurement results of the luminescence characteristics when a direct current voltage is applied to the manufactured organic EL elements.

### [Example 16]

Organic EL elements were manufactured under the same conditions except that the Compound (166) obtained in Example 5 was used instead of Compound (33) in Example 1 as the capping layer 9 in Example 12. The properties of the manufactured organic EL elements were measured in the air and at room temperature and Table 3 summarizes and shows the measurement results of the luminescence characteristics when a direct current voltage is applied to the manufactured organic EL elements.

### [Example 17]

Organic EL elements were manufactured under the same conditions except that the Compound (167) obtained in Example 6 was used instead of Compound (33) in Example 1 as the capping layer 9 in Example 12. The properties of the manufactured organic EL elements were measured in the air and at room temperature and Table 3 summarizes and shows the measurement results of the luminescence characteristics when a direct current voltage is applied to the manufactured organic EL elements.

### [Example 18]

Organic EL elements were manufactured under the same conditions except that the Compound (168) obtained in Example 7 was used instead of Compound (33) in Example 1 as the capping layer 9 in Example 12. The properties of the manufactured organic EL elements were measured in the air and at room temperature, and Table 3 summarizes and shows the measurement results of the luminescence characteristics when a direct current voltage is applied to the manufactured organic EL elements.

### [Example 19]

Organic EL elements were manufactured under the same conditions except that the Compound (169) obtained in Example 8 was used instead of Compound (33) in Example 1 as the capping layer 9 in Example 12. The properties of the manufactured organic EL elements were measured in the air and at room temperature and Table 3 summarizes and shows the measurement results of the luminescence characteristics when a direct current voltage is applied to the manufactured organic EL elements.

### [Example 20]

Organic EL elements were manufactured under the same conditions except that the Compound (170) obtained in Example 9 was used instead of Compound (33) in Example 1 as the capping layer 9 in Example 12. The properties of the manufactured organic EL elements were measured in the air and at room temperature and Table 3 summarizes and shows the measurement results of the luminescence characteristics when a direct current voltage is applied to the manufactured organic EL elements.

### [Comparative Example 1]

For comparison, an organic EL element was manufactured under the same conditions except that Alq₃ was used instead of Compound (33) in Example 1 as the capping layer 9 in Example 12. The properties of the manufactured organic EL elements were measured in the air and at room temperature and Table 3 summarizes and shows the measurement results of the luminescence characteristics when a direct current voltage is applied to the manufactured organic EL elements.

### [Comparative Example 2]

For comparison, an organic EL element was manufactured under the same conditions except that Compound (CPL-1) was used instead of Compound (33) in Example 1 as the capping layer 9 in Example 12. The properties of the manufactured organic EL elements were measured in the air and at room temperature and Table 3 summarizes and shows the measurement results of the luminescence characteristics when a direct current voltage is applied to the manufactured organic EL elements.

The results of measuring element lifetimes using the organic EL elements manufactured in the Examples and Comparative Examples are summarized and shown in Table 3. The element lifetimes measured in the present invention was measured as the time required for the luminance to decay to 95% of its initial value under constant current driving at 10 mA/cm².

**[Table 3]**

| | Capping layer | Voltage [V] (@10 mA/cm²) | Luminance [cd/m²] (@10 mA/cm²) | Emission efficiency [cd/A] (@10 mA/cm²) | Power efficiency [Im/W] (@10 mA/cm²) | Element lifetime for decay to 95% |
|---|---|---|---|---|---|---|
| Example 12 | Compound (33) | 3.65 | 825 | 8.25 | 7.11 | 156 hours |
| Example 13 | Compound (47) | 3.64 | 819 | 8.19 | 7.07 | 155 hours |
| Example 14 | Compound (164) | 3.66 | 812 | 8.12 | 6.97 | 151 hours |
| Example 15 | Compound (165) | 3.65 | 822 | 8.21 | 7.07 | 151 hours |
| Example 16 | Compound (166) | 3.65 | 818 | 8.18 | 7.05 | 159 hours |
| Example 17 | Compound (167) | 3.64 | 809 | 8.09 | 6.99 | 162 hours |
| Example 18 | Compound (168) | 3.64 | 810 | 8.10 | 7.00 | 170 hours |
| Example 19 | Compound (169) | 3.66 | 819 | 8.18 | 7.03 | 167 hours |
| Example 20 | Compound (170) | 3.64 | 821 | 8.21 | 7.09 | 151 hours |
| Comparative Example 1 | Alq₃ | 3.65 | 714 | 7.14 | 6.15 | 114 hours |
| Comparative Example 2 | Compound (CPL-1) | 3.64 | 782 | 7.82 | 6.75 | 141 hours |

As shown in Table 3, the driving voltage when a current with a current density of 10 mA/cm² is applied was nearly identical for the elements of Comparative Examples 1 and 2, and Examples 12 to 20, whereas the elements of the Examples showed significant improvement in terms of luminance, emission efficiency, power efficiency, and element lifetime compared to the elements of the Comparative Examples. This demonstrates that the carbazole compound of the present invention is a material preferably used in a capping layer, and can significantly improve the light extraction efficiency of an organic EL element by increasing the refractive index of the capping layer.

### [INDUSTRIAL APPLICABILITY]

The carbazole compound of the present invention is excellent as a compound preferably used in a capping layer of an organic EL element because it has a high refractive index, can significantly improve light extraction efficiency, and is stable in a thin film state. Furthermore, an organic EL element manufactured using the carbazole compound of the present invention in the material for the capping layer can achieve high efficiency. In addition, the use of the compound of the present invention, which does not exhibit absorption in the blue, green, and red wavelength ranges, respectively, is particularly preferable when clear, bright images are desired due to its high color purity. For example, this element is expected to find application in household phone products and lighting applications.

### [Explanation of reference numerals]

1: Glass substrate
2: Transparent anode
3: Hole injection layer
4: Hole transport layer
5: Light-emitting layer
6: Electron transport layer
7: Electron injection layer
8: Cathode
9: Capping layer

## Claims

1. A carbazole compound represented by the following general formula (A) or (B): 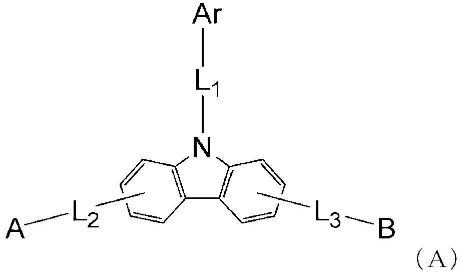 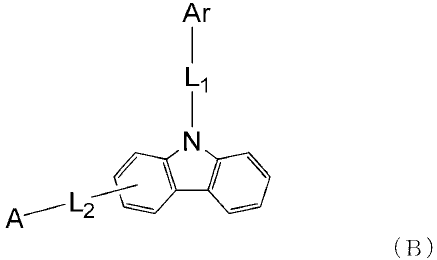 wherein
Ar, A, and B respectively is a substituted or unsubstituted aromatic hydrocarbon group, a substituted or unsubstituted aromatic heterocyclic group, or a substituted or unsubstituted condensed polycyclic aromatic group, with the proviso that at least one of A and B is a substituted or unsubstituted oxazolopyridyl group, or a substituted or unsubstituted oxazolopyrazyl group, and
L₁ to L₃ respectively is a single bond, an unsubstituted divalent aromatic hydrocarbon group, an unsubstituted divalent aromatic heterocyclic group, or an unsubstituted divalent condensed polycyclic aromatic group.

2. The carbazole compound of claim 1, represented by the following formula (C) or (D): 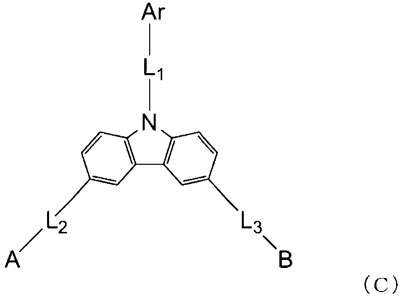 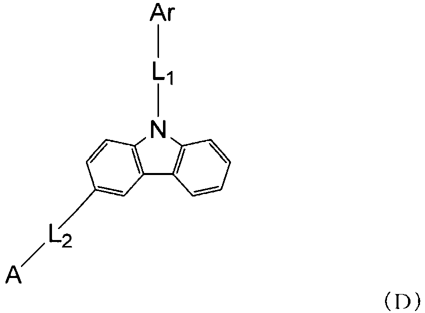 wherein
Ar, A, and B and L₁ to L₃ have the definitions given in the general formula (A) or (B).

3. The carbazole compound of claim 2, wherein L₁ to L₃ in the general formula (C) or (D) are a single bond, an unsubstituted phenylene group, or an unsubstituted naphthylene group.

4. The carbazole compound of claim 3, wherein L₁ to L₃ in the general formula (C) or (D) are a single bond, an unsubstituted 1,4-phenylene group, or an unsubstituted 2,6-naphthylene group.

5. The carbazole compound of claim 4, wherein Ar, A, and B in the general formula (C) or (D) are a substituted or unsubstituted phenyl group, a substituted or unsubstituted naphthyl group, a substituted or unsubstituted quinolyl group, a substituted or unsubstituted isoquinolyl group, a substituted or unsubstituted quinoxalyl group, a substituted or unsubstituted phenanthrenyl group, a substituted or unsubstituted phenantronyl group, a substituted or unsubstituted dibenzofuranyl group, a substituted or unsubstituted dibenzothienyl group, a substituted or unsubstituted benzoxazolyl group, a substituted or unsubstituted benzothiazolyl group, a substituted or unsubstituted benzofuranyl group, a substituted or unsubstituted benzothienyl group, a substituted or unsubstituted oxazolopyridyl group, or a substituted or unsubstituted oxazolopyrazyl group.

6. The carbazole compound of claim 5, wherein Ar in the general formula (C) or (D) are an unsubstituted phenyl group, an unsubstituted naphthyl group, an unsubstituted quinolyl group, an unsubstituted isoquinolyl group, an unsubstituted quinoxalyl group, an unsubstituted phenanthrenyl group, an unsubstituted phenantronyl group, an unsubstituted dibenzofuranyl group, an unsubstituted dibenzothienyl group, an unsubstituted benzoxazolyl group, an unsubstituted benzothiazolyl group, an unsubstituted benzofuranyl group, an unsubstituted benzothienyl group, an unsubstituted oxazolopyridyl group, or an unsubstituted oxazolopyrazyl group.

7. The carbazole compound of claim 6, wherein at least one of A and B in the general formula (C) or (D) is a substituted or unsubstituted 2-oxazolopyridyl group, a substituted or unsubstituted 5-oxazolopyridyl group, or a substituted or unsubstituted 2-oxazolopyrazyl group.

8. The carbazole compound of claim 7, wherein A and B in the general formula (C) or (D) are a substituted or unsubstituted 2-oxazolopyridyl group, a substituted or unsubstituted 5-oxazolopyridyl group, or a substituted or unsubstituted 2-oxazolopyrazyl group.

9. An organic EL element having at least an anode electrode, a hole transport layer, a light-emitting layer, an electron transport layer, a cathode electrode, and a capping layer in that order, wherein the capping layer contains a carbazole compound represented by the general formula (A) or (B) of claim 1.

10. The organic EL element of claim 9, **characterized in that** when the capping layer is formed to a thickness of 30 nm to 120 nm, the refractive index of the capping layer is 1.70 or more when the transmitted light is in the wavelength range of 450 nm to 750 nm.

11. The organic EL element of claim 9, wherein the capping layer is a laminate or mixed layer composed of two or more types of compounds, wherein at least one type of compound is a carbazole compound represented by the general formula (A) or (B).

12. An electronic equipment or electronic element having a pair of electrodes and at least one organic layer sandwiched between them, wherein the organic layer contains a carbazole compound represented by the general formula (A) or (B) of claim 1.
